# EUROPEAN PATENT SPECIFICATION

(11) **EP 1 975 618 B1**
(45) Date of publication and mention of the grant of the patent: **26.10.2011**
(21) Application number: 08006038.7
(22) Date of filing: 28.03.2008
(51) Int. Cl.: G01N 33/543, B01F 13/00

(54) **Sample detecting method and instrument**
Probenerfassungsverfahren und -instrument
Procédé de détection d'échantillon et instrument

(30) Priority: 30.03.2007 JP 2007095561
(43) Date of publication of application: 01.10.2008
(73) Proprietor: FUJIFILM CORPORATION, Minato-ku Tokyo 106-8620 (JP)
(72) Inventor: Takahashi, Kazunori, Ashigarakami-gun Kanagawa (JP)
(74) Representative: HOFFMANN EITLE

(56) References cited:
- EP-A- 0 435 245
- US-A- 5 922 591
- US-A1- 2005 250 129
- US-A1- 2005 287 673

## Description

### BACKGROUND OF THE INVENTION

### Field of the Invention

The present invention relates to a method and an instrument for detecting a sample or analyte, and more particularly to a technique by which bio-related information is obtained by detecting any reaction between a sample and a reagent in a sample liquid in a Micro Total Analysis System (µTAS) for conducting chemical or biochemical analyses and chemical syntheses over a chip.

### Description of the Related Art

Conventionally applied methods of diagnosing immunity include immunochromatography. For instance in Japanese Patent Application Laid-Open No. 2003-83958, a detecting instrument comprising a membrane having a narrow-width long thin film on which a reactant contact region and a detecting region are formed and an absorbing region (water absorbing pad) stuck thereto is proposed. In this detecting instrument, by moving an antigen in a sample liquid to the surface of the membrane by capillarity from the reactant contact region to the absorbing region, an antigen-antibody complex (marker) is formed from the antigen in the sample liquid, a pigment-marked antibody and a capture antibody, and detection is accomplished by visually confirming this marker in the detecting region. In this way, immunochromatography has the advantage of enabling anyone to easily and quickly obtain the result.

By immunochromatography described above, capillarity is retained by using the water absorbing pad positioned downstream of the membrane to absorb the sample liquid. However, since the sample liquid is moved only once in one direction, there is a problem that a large quantity of the antigen remains uncombined with an antibody in the sample liquid. For this reason, accurate detection of the sample in the sample liquid requires a contrivance to maximize the probability for the antigen in the sample liquid to come into contact with an antibody.

Apart from that, there is a method by which a sample liquid and a reagent are mixed in a micro-channel. However, since laminar flows are mainly formed in a micro-space, this is not suitable for actively mixing a sample liquid and a reagent. For instance according to Japanese Patent Application Laid-Open No. 2006-52950, mixing of sample drops and reagent drops is facilitated by oscillating a collection of drops, in which sample drips and reagent drips are combined, linearly in a linear micro-channel.

However, the method proposed in Japanese Patent Application Laid-Open No. 2006-52950 requires incorporation of piezo-elements into a microchip as a device for oscillating the collection of drops in the micro-channel. For this reason, this method involves a problem of being too expensive and therefore unrealistic for application to simple disposable microchips.

### SUMMARY OF THE INVENTION

An object of the present invention, attempted in view of these circumstances, is to provide a method and an instrument for detecting a sample which can fluidize sample liquid without having to use any external liquid driving device and efficiently accomplish various detecting reactions within a small and simple-structured instrument in a short period of time.

In order to achieve the object stated above, according to a first aspect of the invention, there is provided a method of detecting a sample contained in sample liquid, comprising the steps of: a step of injecting the sample liquid into a container having a micro-space and provided with stirring particles in the micro-space; a step of inclining the container into which the sample liquid has been injected at a prescribed angle of inclination with respect to the vertical direction; a step of causing the sample liquid to be fluidized by sedimentation of the stirring particles in the inclined container; and a step of bringing the fluidized sample liquid into contact with a reagent stuck to an inner wall face of the micro-space in advance.

In the foregoing, "inclining the container ... with respect to the vertical direction" means both inclining the container from an upright state with respect to the vertical direction (the direction of gravity) by tilting the container in the thickness direction of the micro-space and inclining the container from a laid state by raising the container in the thickness direction of the micro-space.

According to the first aspect of the invention, the Boycott effect is caused to arise in the inclined micro-space by letting the stirring particles sediment. This Boycott effect cannot only fluidize the sample liquid along the inner wall face of the micro-space in the upper part (e.g. the upper face out of the opposite faces in the thickness direction where the micro-space is rectangular) but also enhance the efficiency of contact with the reagent stuck to the inner wall face of the micro-space.

It may be relevant here to state that the behavior of the stirring particles within the micro-space varies with the direction in which the container is erected with respect to the direction of gravity. The stirring particles in the container vertically erected move in the vertical direction. As the sample liquid moves in a direction reverse to gravity at the same time and the stirring particles and the sample liquid collide with each other head on, the flow of the sample liquid is susceptible to obstruction by the stirring particles. According to the invention, unlike this behavior, the stirring particles settle down in the direction of gravity in the micro-space within the inclined container, and the sample liquid rises along the inner wall face of the micro-space in the upper part (the upward flow of the sample liquid moves closer to the wall face than the middle of the micro-space in the widthwise direction). This makes it possible for the sedimenting direction of the stirring particles and the rising direction of the sample liquid to avoid becoming exactly reverse to each other. This makes it difficult for the two flows to interfere with each other and enables the sample liquid to efficiently fluidize.

In this way, by causing the Boycott effect to arise by utilizing the stirring particles in the micro-space, the flow of the sample liquid along the inner wall face of the micro-space can be formed without having to use any external liquid driving device such as a pump. The reaction can be accelerated by enhancing the efficiency of contact between the sample and the reagent stuck to the inner wall face of the micro-space in advance.

According to a second aspect of the invention, the angle of inclination is 30° to 60° with respect to the vertical direction in the configuration according to the first aspect.

The second aspect concerns a suitable angle inclination of the container when the Boycott effect is applied to the micro-space, wherein the angle of inclination preferably is in the range of 30° to 60°, more preferably 45°. This is because the flow velocity is at highest when the angle of inclination is 45° (Reference: an article by S. Kaihara and A. Sakanashi, p.72, journal Biorheology (in Japanese), first issue published on June 4, 1999 by Yoneda Shuppan), but the same approximately holds true in the range of 30° to 60° though there is some difference.

According to a third aspect of the invention, the flow velocity of the sample liquid is regulated with any one or more of a specific gravity, a grain size and a volume fraction to the sample liquid, of the stirring particles in the configuration according to the first or second aspect.

According to a fourth aspect of the invention, the stirring particles are provided with magnetic particles and the sedimentation velocity of the stirring particles in the container is regulated by controlling a magnetic field applied to the stirring particles in the configuration according to any one of the first through third aspects.

In this way, by varying the magnetic field applied to the stirring particles having magnetic particles, the sedimentation velocity of the stirring particles in the micro-space can be regulated, and the flow of the sample liquid caused by the Boycott effect can be thereby regulated.

According to a fifth aspect of the invention, the sedimentation of the stirring particles is repeated by turning upside down the micro-space in the lengthwise direction in the configuration according to any one of the first through fourth aspects.

When the stirring particles having completed sedimentation in the container accumulate on the bottom of the container, the Boycott effect can no longer be continued. According to the fifth aspect of the invention, even if the stirring particles accumulate on the bottom of the container and the sample liquid can no longer be fluidized, the Boycott effect can be continued by turning the container upside down and inclining it again. In this way, even if any unreacted sample remains in the sample liquid, by turning the container upside down to cause the stirring particles to sediment, the sample in the sample liquid and the reagent stuck in the container can be efficiently brought into contact with each other.

According to a sixth aspect of the invention, the sample in the sample liquid is an antigen and the reagent is an antibody specifically reacting with the sample, and whereby an antigen-antibody reaction is caused to occur in the configuration according to any one of the first through fifth aspects.

The sixth aspect represents a preferred mode for implementing the invention, which enables tests using an antigen-antibody reaction to be accomplished easily with high sensitivity.

In order to achieve the object stated above, according to a seventh aspect of the invention, there is provided an instrument for detecting a sample contained in sample liquid, comprising: a container having a micro-space, to whose inner wall face a reagent is stuck in advance; an inclining device having a base which supports a supporting member on which the container is fitted, wherein the inclining device is arranged to incline the container at a prescribed angle with respect to the vertical direction; and stirring particles to be inputted to the container.

According to the seventh aspect, by loading sample liquid into a container loaded with stirring particles and inclining the container, for instance by hand, with respect to the vertical direction, the Boycott effect can be caused to occur. In this way, the sample liquid can be fluidized and the sample in the sample liquid and the reagent stuck in the inner wall face of the micro-space can be efficiently brought into contact with each other without having to use any external liquid driving device. Therefore, detecting a reaction in a small container can be caused to take place accurately in a short period of time.

According to an eighth aspect of the invention, the micro-space has a rectangular solid shape in the configuration according to the seventh aspect.

This enables the sedimenting distance of the stirring particles to be uniformized in the widthwise direction and there a stable flow to be formed.

According to a ninth aspect of the invention, the container is transparent in the configuration according to the seventh or eighth aspect.

The use of a transparent container makes possible visual perception of the point of time when the reaction between the sample in the sample liquid and the reagent stuck to the wall face of the micro-space. As the state of the upward flow of the sample liquid can also be visually perceived, the container can be adjusted to an appropriate angle of inclination. As the material of the container, a transparent resin such as acryl, quartz glass or Pyrex can be used.

According to a 10th aspect of the invention, the stirring particles are polystyrene particles in the configuration according to any one of the seventh through ninth aspects.

According to an 11th aspect of the invention, the sample in the sample liquid is an antigen and the reagent is an antibody specifically reacting with the sample in the configuration according to any one of the seventh through 10th aspects.

The sample detecting method and detecting instrument according to the invention enable sample liquid to be fluidized without having to use any external liquid driving device and various detecting reactions to be efficiently accomplished within a small and simple-structured instrument in a short period of time.

### BRIEF DESCRIPTION OF THE DRAWINGS

Fig. 1 is a conceptual diagram showing one example of detecting instrument according to the present invention;
Fig. 2 shows a perspective view of the detecting instrument of Fig. 1 in an inclined state;
Figs. 3A, 3B and 3C illustrate the procedure of a sample detecting method according to the invention;
Figs. 4A and 4B illustrate the fluid conditions of the sample liquid when the erected state of the container is varied;
Fig. 5 is a schematic sectional view showing a state in which the top and bottom of the container are reversed in the lengthwise direction; and
Fig. 6 is a conceptual diagram showing another example of detecting instrument according to the invention.

### DETAILED DESCRIPTION OF THE PREFERRED EMBODIMENTS

Sample detecting methods and instruments which are preferred embodiments of the present invention will be described in detail below with reference to the accompanying drawings.

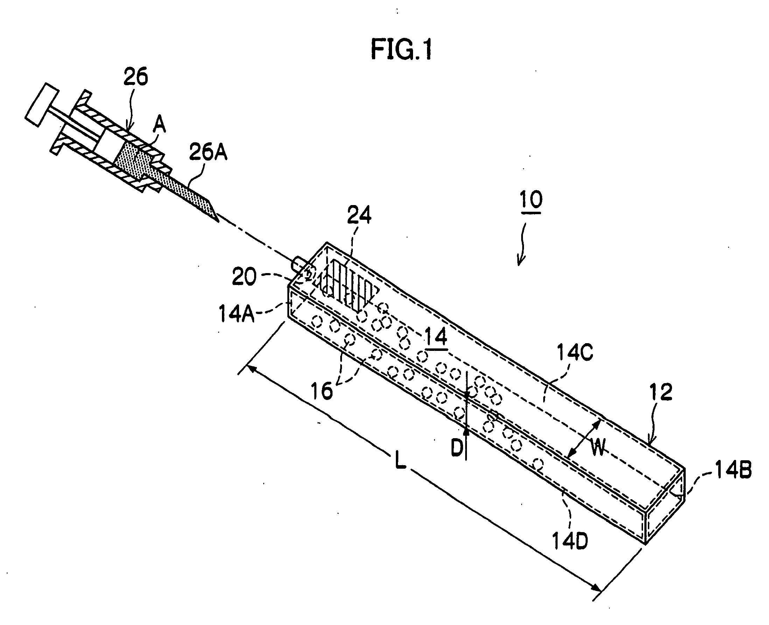

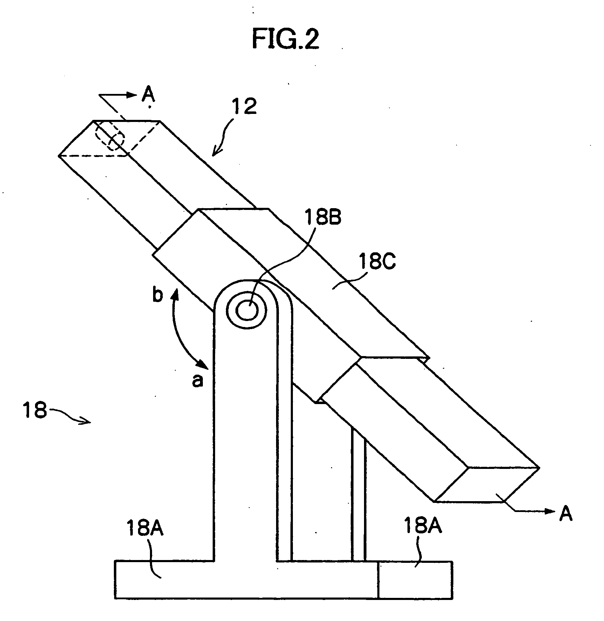

Fig. 1 is a conceptual diagram showing one example of detecting instrument 10 according to the invention, and Fig. 2, a perspective view of the detecting instrument 10 of Fig. 1 in an inclined state.

As shown in Fig. 1, the detecting instrument 10 according to the invention mainly comprises a container 12 in which a reagent is stuck inside in advance and beads 16 (stirring particles) filling a micro-space 14 within the container 12.

Within the container 12, the small-thickness cuboidal micro-space 14 is formed. The thickness D of the micro-space 14 is preferably between 50 µm and 2 mm, and more preferably between 100 µm and 500 µm. Preferably, the length L of the micro-space 14 is to be set relative to the thickness D such that the aspect ratio represented by L/D falls in a range of 5 to 50, where D is the thickness and L is the length of the micro-space 14. For instance, where the thickness D of the micro-space 14 is 50 µm, the length L is set between 250 µm and 2.5 mm, or where the thickness D of the micro-space 14 is 2 mm, the length L is set between 10 mm and 100 mm.

Therefore, the size of the micro-space 14 can be so set as to satisfy these conditions correspondingly to the sample quantity required for each of various examinations. It is preferable for the width W of the micro-space 14, though not specifically limited, to be in approximately the same range as the thickness D. Although the micro-space 14 is supposed to be cuboidal in this embodiment, this is not the only shape to be chosen, but it may as well be cylindrical or the like only if it has a certain length.

The shape of the container 12 is not limited to what is shown in Fig. 1, but any structure having the micro-space 14 is acceptable. For instance, the structure may have the micro-space 14 in the middle of the substrate.

A loading inlet 20 through which sample liquid A is to be loaded is formed at one end of the container 12 in the lengthwise direction. This loading inlet 20 is sealed with a sealing member (not shown) made of rubber or the like, and a needle 26A of a syringe 26 (see Fig. 1) as a loading device can be pierced and withdrawn through this sealing member. Out of the (six) faces constituting the cuboidal micro-space 14, the face toward the loading inlet 20 will hereinafter be referred to as a ceiling 14A, the one face opposite it, as a bottom 14B. In the following description, the face of the micro-space 14 located at a higher position in the vertical direction when the container 12 is inclined will be referred to as an upper face 14C, and the face opposite it, as a lower face 14D.

An air escape (not shown) is formed in the upper face 14C of the micro-space 14. A testing reagent 24 is stuck in advance to the upper part of the upper face 14C of the micro-space 14. As the probability of the presence of the beads 16 is higher toward the lower part of the upper face 14C of the micro-space 14, it is preferable to stick the testing reagent 24 only to the upper part of the upper face 14C of the micro-space 14. However, the testing reagent 24 may be stuck to substantially the whole of the upper face 14C.

As the testing reagent 24, an antibody can be suitably used. By bringing an antigen in the sample liquid A into reaction with the antibody stuck to the upper part of the upper face 14C of the micro-space 14, a test by so-called enzyme-linked immunosorbent assay (ELISA) can be conducted.

A sandwich ELISA is a simple testing method utilizing an antigen-antibody reaction using a second antibody, which is obtained by fixing the antibody to the antigen to be examined and is marked with fluorescence, a pigment, a radioactive substance, an enzyme or the like. Since the antigen need not be directly marked, the reaction can be easily detected with high sensitivity. The testing reagent 24 is not limited to the antibody, but may be a reagent which permits some test or another.

A suitable method of sticking the testing reagent 24 to the upper part of the upper face 14C of the micro-space 14 is, for example, to eject the testing reagent 24 from the nozzle of an ink jet device and spray it onto the upper part of the upper face 14C of the micro-space 14, but this is not the only available method.

It is preferable for the grain size of the beads 16 loaded into the micro-space 14 to be about 1 to 1000 µm, more preferably 1 to 50 µm. The specific gravity of the beads 16 is preferably 1 to 1.5, more preferably 1 to 1.2. It is preferable for the loading ratio of the beads 16 to be set to 50 v/v% as their volume fraction to the quantity of the sample liquid loaded into the micro-space 14.

Although, there is no limitation regarding the material for the beads 16 if it does not take part in the immunoreaction of the sample liquid (inactive material), it should preferably satisfy the grain size and specific gravity requirements mentioned above. Materials for such beads 16 include polystyrene and latex for instance. Further, by applying surface modification to the beads 16 in one or another way, for instance forming polyethylene glycol or the like on the surface, they may be insensitive to the sample liquid or its reaction.

By regulating the grain size, specific gravity and loading ratio of the beads 16 in this way, the sedimentation velocity of the beads 16 in the micro-space 14 can be controlled, and therefore the flow rate of the sample liquid can also be controlled.

This detecting instrument 10 can be inclined with an inclining device 18 shown in Fig. 2 for instance. As shown in Fig. 2, the inclining device 18 is configured as to have the strut part of a base 18A support a supporting member 18C via a swinging mechanism 18B in the directions of arrow a-b. The supporting member 18C is a cylinder having a guiding mechanism (not shown) inside, allowing the container 12 to be fitted in and pulled out as desired. The swinging mechanism 18B may have any configuration that enables the container 12 supported by the supporting member 18C to remain immovable at a prescribed angle of inclination.

A micromachining technique can be suitably used for fabricating the container 12 having the micro-space 14 as described above. Available micromachining techniques included the following:
(1) LIGA combining X-ray lithography and electroplating;
(2) High aspect ratio photolithography using EPON SU8;
(3) Mechanical micro-cutting (including micro-drilling with a drill whose diameter is in a micro-order);
(4) High aspect ratio machining of silicon using deep RIE;
(5) Hot embossing;
(6) Photo-fabrication;
(7) Laser machining; and
(8) Ion beam process.

Materials that can be suitably used for fabricating the container 12 include metals, glass, ceramics, plastics, silicon and Teflon that can meet heat-resistance, pressure-resistance, solvent-resistance and workability requirements, and polystyrene resin, PMMA resin, quartz glass and Pyrex are particularly preferable. Further, the container 12 is preferably fabricated of a transparent material, because the tested reaction state can be visually perceived.

The sample liquid containing the sample may be, for instance, blood, urine, saliva or the like, though there is no particular limitation.

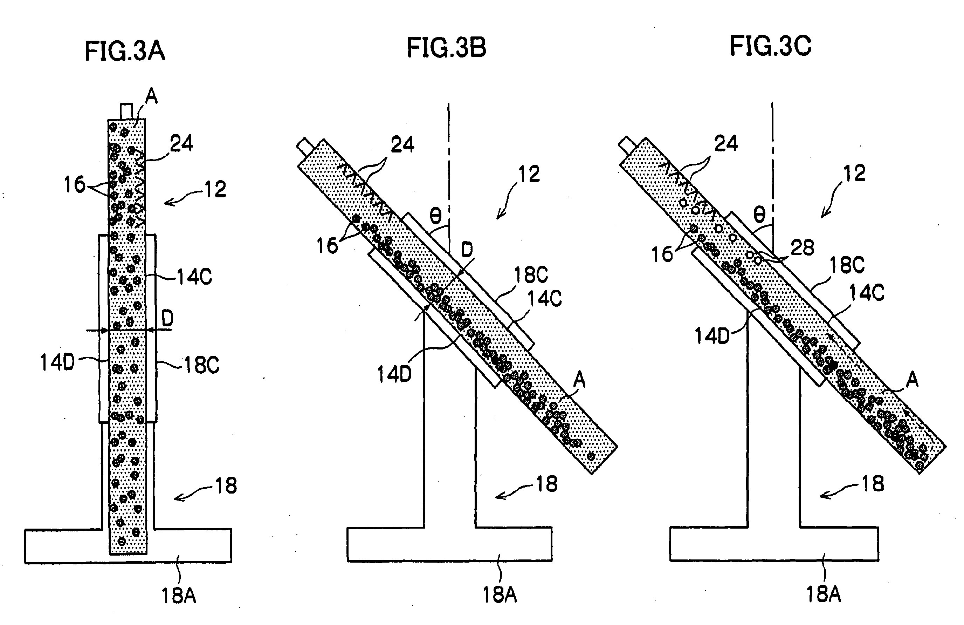

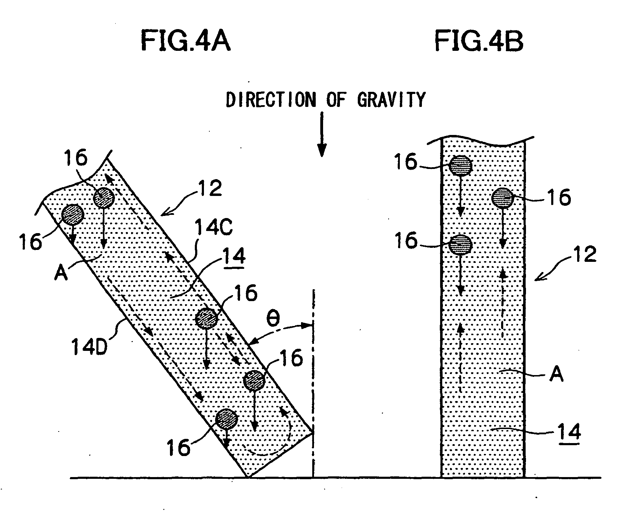

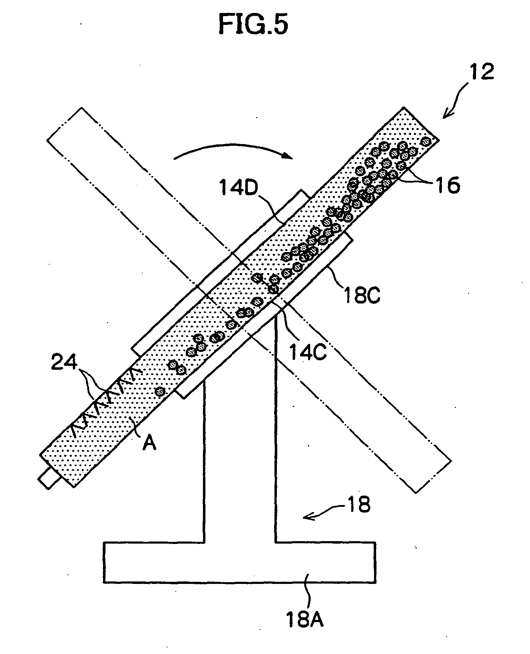

The procedure of detecting the sample in the sample liquid according to the invention by using the detecting instrument 10 configured as described above will now be described with reference to Figs. 3A to 3C through Fig. 5. Figs. 3A to 3C illustrate the procedure of sample detection according to the invention; and Figs. 4A and 4B illustrate the fluid conditions of the sample liquid when the erected state of the container 12 is varied. The sections shown in Figs. 3A to 3C are along line A-A in Fig. 2. Fig. 4A shows the fluid conditions of the sample liquid and the beads 16 when the container 12 is inclined, and Fig. 4B, the fluid conditions of the sample liquid and the beads 16 when the container 12 is erected upright. Fig. 5 is a schematic sectional view showing a state in which the top and bottom of the container 12 are reversed in the lengthwise direction.

First, as shown in Fig. 3A, the container 12 held by the inclining device 18 is placed in an upright state. Then, the micro-space 14 in the container 12 is filled with the sample liquid A by using the syringe 26 shown in Fig. 1.

Next, as shown in Fig. 3B, the container 12 is inclined at an angle of inclination of 30 to 60°, preferably 45°, with respect to the vertical direction (the direction of gravity). In this process, the container 12 is so inclined as to position the upper face 14C of the micro-space 14 upward in the vertical direction. The action to incline the container 12 may precede or follow the loading of the sample liquid A into the container 12. The container 12 need not be filled with the sample liquid A in the upright state, but may instead be erected to the angle of inclination of 30 to 60° after it is filled with the liquid in a laid state.

Then, as further shown in Fig. 3B, the Boycott effect is caused to arise in the micro-space 14 by letting the beads 16 naturally sediment in a state in which the container 12 is inclined. Thus, as shown in Fig. 4A, while the beads 16 sediment in the direction of gravity, the sample liquid A rises along the upper face 14C of the micro-space 14 (the inner wall face of the container in its upper part). As a result, as shown in Fig. 4A, a circular flow of the sample liquid A (the dotted arrow in Fig. 4A) in which the upward flow rising along the upper face 14C of the micro-space 14 falls as a downward flow along the lower face 14D of the micro-space 14 is formed in the micro-space 14.

Then, as shown in Fig. 3C, as the sedimentation of the beads 16 and the upward flow of the sample liquid A never collide head on, it is made possible to quickly bring into contact a sample 28 contained in the sample liquid and the testing reagent 24 stuck to the upper face 14C of the micro-space 14.

On the other hand, if the beads 16 are let naturally sediment in a state in which the container 12 is in an upright state, as the sedimentation of the beads 16 and the upward flow of the sample liquid A collide head on as shown in Fig. 4B, the upward flow of the sample liquid A becomes more susceptible to obstruction.

Incidentally, the Boycott effect is discussed in detail in A.E. Boycott, "Sedimentation of blood corpuscles", Nature (London), 104, 532, 1920) and an article by S. Kaihara and A. Sakanashi, p.72, journal Biorheology (in Japanese), first issue published on June 4, 1999 by Yoneda Shuppan).

Even if the sample 28 in the sample liquid A fails to come into contact with the testing reagent 24 and remains unreacted, fluidization of the sample liquid A can be caused to resume by moving the swinging mechanism 18B and thereby turning the container 12 upside down as shown in Fig. 5.

By causing the Boycott effect to arise in the micro-space 14 by using the beads 16 in this way, an upward flow of the sample liquid along the upper face 14C of the micro-space 14 can be formed without having to use any external liquid driving device such as a pump. This enables the effect to stir the sample liquid to be exerted, and the probability for components in the sample liquid A to come into contact with the upper face of the micro-space 14 to be dramatically enhanced. Therefore, by sticking the testing reagent 24 to the upper part of the upper face 14C of the micro-space 14 in advance, the efficiency of contact between the sample 28 of the sample liquid and the testing reagent 24 can be improved to enable testing to be accurately carried out in such a simple space as the micro-space 14.

Further by turning the container 12 upside down as shown in Fig. 5, the fluidization of the sample liquid A can be continued by causing the beads 16 to sediment again in the container 12. This enables the sample in the sample liquid to react highly efficiently.

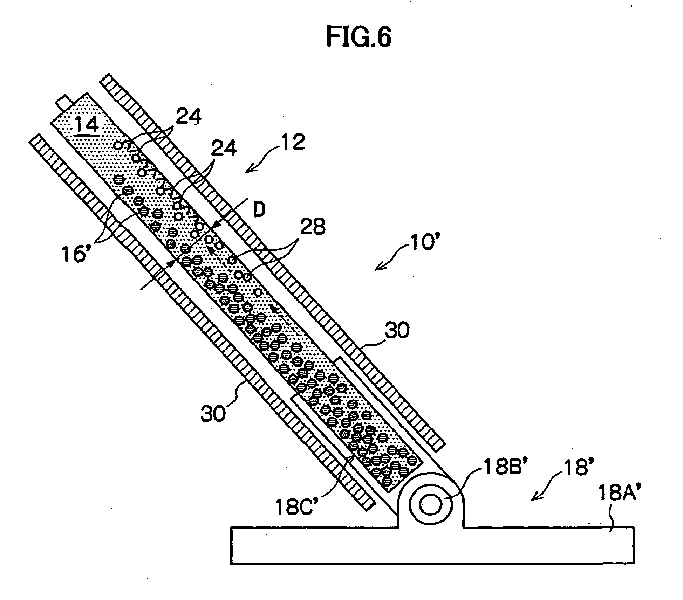

Fig. 6 is a conceptual diagram showing another example of detecting instrument according to the invention.

As shown in Fig. 6, a detecting instrument 10' is configured substantially similar to that shown in Fig. 1 except that magnetic field applying devices 30 and 30 are disposed outside the container 12 and magnetic beads 16' are used as the beads 16. Further in Fig. 5, what is differently shaped from its counterpart in Fig. 2 is used as an inclining device 18'. Members having the same functions as their respective counterparts in Fig. 1 are assigned the same reference numerals and characters, and their detailed description will be omitted.

The inclining device 18' is composed by swingably supporting the supporting member 18C on the central part of the upper face of the base 18A via the swinging mechanism 18B. The supporting member 18C is so composed as to have a concave section, into which the lower part of the container 12 detachably snapped.

The magnetic field applying devices 30 and 30 are so arranged as to oppose the upper face 14C of the micro-space 14 and the lower face 14D, respectively. As these magnetic field applying devices 30, there is no particular limitation, and any devices that can apply magnetic fields to the magnetic beads 16' in the micro-space 14 can be used, such as magnets or electromagnetic coils for instance.

As the magnetic beads 16', magnetic particles whose surface is coated with a polymer layer insensitive to the sample liquid A can be used. Available materials for the magnetic particles and the polymer layer include, for instance, iron oxide and polyethylene glycol (PEG), respectively.

By applying a magnetic field to the magnetic beads 16' in the micro-space 14 in this configuration, the sedimentation velocity of the magnetic beads 16' can be regulated, and the flow rate of the sample liquid A can be thereby regulated.

Whereas sample detecting methods and instruments which are preferred embodiments of the present invention have been described so far, the invention is not limited to these embodiments, but can be implemented in various other modes.

For instance, though the sample liquid A is supposed to be injected into the container 12 loaded in advance with the beads 16 in the embodiments described above, the sequence is not limited to this, but the sample liquid and the beads 16 may as well be mixed in advance and then loaded into the container 12.

Further, though the device for inclining the container 12 was described with regard to the foregoing embodiments in the forms of the inclining device 18 in Fig. 2 and the inclining device 18' in Fig. 5, these are not the only options, but the container 12 in a hand-held state may be inclined with respect to the vertical direction. Example

Next, as a specific example of implementing the invention, a method of detecting an antigen contained in urine by immunochromatography in a sandwich process using the detecting instrument 10 shown in Fig. 1 will be described.

In the container 12 made of transparent polystyrene used in this example, the micro-space 14 measuring 0.5 mm in thickness, 1 mm in width and 40 mm in length was formed. The testing reagent 24 with a visualizing marker which specifically couples with the target antigen contained in urine was stuck toward the upper part of the upper face 14C of the micro-space 14 in advance.

The testing reagent 24 was stuck toward the upper part of the upper face 14C of the micro-space 14 by ejecting the testing reagent (antibody) from the ink ejecting nozzle of an ink jet device.

Further, polystyrene particulates of 10 µm in grain size and 1.05 g/cm³ in specific gravity were loaded into the container 12 as the beads 16. The polystyrene particulates were loaded to a volume fraction of 50 v/v% to the urine to be loaded. About 10 µL of urine was loaded into the container 12.

Next, as shown in Fig. 3B, the container 12 was inclined by 45° with respect to the vertical direction (the direction of gravity) and kept it still.

As a result, the polystyrene particulates gradually sedimented in the micro-space 14 and, three minutes after the container 12 was inclined, an upward flow of urine along the vicinities of the upper face 14C of the micro-space 14 (within 50 µm from the upper face 14C) was confirmed. The flow rate of the urine then was measured with a particle image velocimetry (PIV) system, and determined to be 20 µm/second.

As the urine was fluidized along the upper face C of the micro-space 14, the antigen in the urine came into contact with the antibody having the visualizing marker (gold colloid particles) stuck to the upper face C and accelerated the antigen-antibody reaction.

As a result, the visualizing marker was fixed to the upper face C of the micro-space 14 through the antigen-antibody coupling. The fixed gold colloid was confirmed to have a density not less than certain level, and the quantity of the antigen in the urine was found determinable by the gradation of the gold colloid-derived color. By optically measuring this gold colloid, quantitative measurement was made possible.

## Claims

1. A method of detecting a sample (28) contained in sample liquid, comprising the steps of:
a step of injecting the sample liquid into a container (12) having a micro-space (14) and provided with stirring particles (16) in the micro-space (14);
a step of inclining the container (12) into which the sample liquid has been injected at a prescribed angle of inclination with respect to the vertical direction;
a step of causing the sample liquid to be fluidized by sedimentation of the stirring particles (16) in the inclined container (12); and
a step of bringing the fluidized sample liquid into contact with a reagent (24) stuck to an inner wall face of the micro-space (14) in advance.

2. The sample detecting method according to Claim 1, wherein the angle of inclination is 30° to 60° with respect to the vertical direction.

3. The sample detecting method according to Claim 1 or 2, wherein the flow velocity of the sample liquid is regulated with any one or more of a specific gravity, a grain size and a volume fraction to the sample liquid, of the stirring particles (16).

4. The sample detecting method according to any one of Claims 1 through 3, wherein the stirring particles (16) are provided with magnetic particles (16') and the sedimentation velocity of the stirring particles (16) in the container (12) is regulated by controlling a magnetic field applied to the stirring particles (16).

5. The sample detecting method according to any one of Claims 1 through 4, wherein the sedimentation of the stirring particles (16) is repeated by turning upside down the micro-space (14) in the lengthwise direction.

6. The sample detecting method according to any one of Claims 1 through 5, wherein the sample (28) in the sample liquid is an antigen and the reagent (24) is an antibody specifically reacting with the sample (28), and whereby an antigen-antibody reaction is caused to occur.

7. An instrument (10, 10') for detecting a sample (28) contained in sample liquid, comprising:
a container (12) having a micro-space (14), to whose inner wall face a reagent (24) is stuck in advance;
an inclining device (18) having a base (18A) which supports a supporting member (18C) on which the container (12) is fitted, wherein the inclining device (18) is arranged to incline the container (12) at a prescribed angle of inclination with respect to the vertical direction; and
stirring particles (16) inputted to the container (12).

8. The detecting instrument according to Claim 7, wherein the micro-space (14) has a rectangular solid shape.

9. The detecting instrument according to Claim 7 or 8, wherein the container (12) is transparent.

10. The detecting instrument according to any one of Claims 7 through 9, wherein the stirring particles (16) are polystyrene particles.

11. The detecting instrument according to any one of Claims 7 through 10, wherein the sample (28) in the sample liquid is an antigen and the reagent (24) is an antibody specifically reacting with the sample (28).

## Patentansprüche

1. Verfahren zum Nachweis einer Probe (28), die in einer Probenflüssigkeit enthalten ist, welches folgende Schritte aufweist:
Einspritzen der Probenflüssigkeit in einen einen Mikroraum (14) aufweisenden Behälter (12), der mit Durchmischungspartikeln (16) in dem Mikroraum (14) versehen ist;
Neigen des Behälters (12), in den die Probenflüssigkeit eingespritzt worden ist, um einen bestimmten Neigungswinkel bezüglich der vertikalen Richtung;
Bewirken, dass die Probenflüssigkeit durch Sedimentierung der Durchmischungspartikel (16) in dem geneigten Behälter (12) verwirbelt wird; und
in Kontakt-Bringen der verwirbelten Probenflüssigkeit mit einem Reagenz (24), das vorher an einer inneren Wandfläche des Mikroraums (14) befestigt worden ist.

2. Verfahren zum Probennachweis nach Anspruch 1, bei dem der Neigungswinkel bei 30° bis 60° bezüglich der vertikalen Richtung liegt.

3. Verfahren zum Probennachweis nach Anspruch 1 oder 2, bei dem die Fließgeschwindigkeit der Probenflüssigkeit durch eine oder mehrere Eigenschaften der Durchmischungspartikel (16), ausgewählt aus einer Gruppe bestehend aus einer spezifischen Dichte, einer Korngröße und einem Volumenanteil zu der Probenflüssigkeit, reguliert wird.

4. Verfahren zum Probennachweis nach einem der Ansprüche 1 bis 3, bei dem die Durchmischungspartikel (16) mit magnetischen Partikeln (16') versehen sind und die Sedimentierungsgeschwindigkeit der Durchmischungspartikel (16) in dem Behälter (12) durch Steuern eines magnetischen Felds, das an die Durchmischungspartikel (16) angewendet wird, reguliert wird.

5. Verfahren zum Probennachweis nach einem der Ansprüche 1 bis 4, bei dem die Sedimentierung der Durchmischungspartikel (16) durch Umdrehen des Mikroraums (14) in die Längsrichtung wiederholt wird.

6. Verfahren zum Probennachweis nach einem der Ansprüche 1 bis 5, bei dem die Probe (28) in der Probenflüssigkeit ein Antigen ist und das Reagenz (24) ein Antikörper ist, der spezifisch mit der Probe (28) reagiert, und wobei bewirkt wird, dass eine Antigen-Antikörper-Reaktion stattfindet.

7. Vorrichtung (10, 10') zum Nachweis einer Probe (28), die in einer Probenflüssigkeit enthalten ist, mit:
einem Behälter (12) mit einem Mikroraum (14), an dessen innerer Wandfläche vorher ein Reagenz (24) befestigt worden ist;
einem Neigungsbauteil (18) mit einer Basis (18A), die ein Stützelement (18C) stützt, an das der Behälter (12) gefügt ist, wobei das Neigungsbauteil (18) so angeordnet ist, dass der Behälter (12) um einen bestimmten Neigungswinkel bezüglich der vertikalen Richtung geneigt ist; und
Durchmischungspartikeln (16), die in den Behälter (12) eingegeben worden sind.

8. Nachweisvorrichtung nach Anspruch 7, bei welcher der Mikroraum (14) eine rechteckige, feste Form aufweist.

9. Nachweisvorrichtung nach Anspruch 7 oder 8, bei welcher der Behälter (12) transparent ist.

10. Nachweisvorrichtung nach einem der Ansprüche 7 bis 9, bei der die Durchmischungspartikel (16) Polystyrenpartikel sind.

11. Nachweisvorrichtung nach einem der Ansprüche 7 bis 10, bei der die Probe (28) in der Probenflüssigkeit ein Antigen ist und das Reagenz (24) ein Antikörper ist, der spezifisch mit der Probe (28) reagiert.

## Revendications

1. Procédé de détection d'un échantillon (28) contenu dans un liquide d'échantillon, comprenant les étapes de :
une étape d'injection du liquide d'échantillon dans un conteneur (12) ayant un micro-espace (14) et muni de particules de brassage (16) dans le micro-espace (14) ;
une étape d'inclinaison du conteneur (12), dans lequel le liquide d'échantillon a été injecté, à un angle d'inclinaison prescrit par rapport à la direction verticale ;
une étape consistant à amener le liquide d'échantillon à être fluidifié par sédimentation des particules de brassage (16) dans le conteneur incliné (12) ; et
une étape consistant à amener le liquide d'échantillon fluidifié en contact avec un réactif (24) collé à une face formant paroi intérieure du micro-espace (14) à l'avance.

2. Procédé de détection d'échantillon selon la revendication 1, dans lequel l'angle d'inclinaison est de 30° à 60° par rapport à la direction verticale.

3. Procédé de détection d'échantillon selon la revendication 1 ou 2, dans lequel la vitesse d'écoulement du liquide d'échantillon est régulée par l'un quelconque ou plusieurs d'un poids volumique, d'une taille de grain et d'une fraction de volume par rapport au liquide d'échantillon, des particules de brassage (16).

4. Procédé de détection d'échantillon selon l'une quelconque des revendications 1 à 3, dans lequel les particules de brassage (16) sont munies de particules magnétiques (16') et la vitesse de sédimentation des particules de brassage (16) dans le conteneur (12) est régulée en maîtrisant un champ magnétique appliqué aux particules de brassage (16).

5. Procédé de détection d'échantillon selon l'une quelconque des revendications 1 à 4, dans lequel la sédimentation des particules de brassage (16) est répétée en mettant le micro-espace (14) sens dessus dessous dans la direction de la longueur.

6. Procédé de détection d'échantillon selon l'une quelconque des revendications 1 à 5, dans lequel l'échantillon (28) dans le liquide d'échantillon est un antigène et le réactif (24) est un anticorps réagissant spécifiquement avec l'échantillon (28), et de sorte qu'une réaction antigène-anticorps est amenée à se produire.

7. Instrument (10, 10') pour détecter un échantillon (28) contenu dans un liquide d'échantillon, comprenant :
un conteneur (12) ayant un micro-espace (14), un réactif (24) étant collé à l'avance à une face formant paroi intérieure de celui-ci;
un dispositif d'inclinaison (18) ayant une base (18A) qui soutien un élément de soutien (18C) sur lequel le conteneur (12) est ajusté, dans lequel le dispositif d'inclinaison (18) est agencé pour incliner le conteneur (12) à un angle d'inclinaison prescrit par rapport à la direction verticale ; et
des particules de brassage (16) entrées dans le conteneur (12).

8. Instrument de détection selon la revendication 7, dans lequel le micro-espace (14) a une forme pleine rectangulaire.

9. Instrument de détection selon la revendication 7 ou 8, dans lequel le conteneur (12) est transparent.

10. Instrument de détection selon l'une quelconque des revendications 7 à 9, dans lequel les particules de brassage (16) sont des particules de polystyrène.

11. Instrument de détection selon l'une quelconque des revendications 7 à 10, dans lequel l'échantillon (28) dans le liquide d'échantillon est un antigène et le réactif (24) est un anticorps réagissant spécifiquement avec l'échantillon (28).
